# EUROPEAN PATENT APPLICATION

(11) **EP 3 805 355 A1**
(43) Date of publication of application: **14.04.2021**
(21) Application number: 19811622.0
(22) Date of filing: 11.01.2019
(51) Int. Cl.: C12M 1/00, C12M 1/32

(54) **MULTI-LAYER CULTURE VESSEL OBSERVATION SYSTEM, CARRIAGE DEVICE, AND MULTI-LAYER CULTURE VESSEL OBSERVATION DEVICE**

(30) Priority: 30.05.2018 JP 2018103807
(71) Applicant: Shikoku Instrumentation Co., Ltd., Kagawa, 764-0026 (JP)
(72) Inventor: TANABE, Hiroyuki, Nakatado-gun, Kagawa 764-0026 (JP); KAWATA, Yasutomi, Nakatado-gun, Kagawa 764-0026 (JP); ONO, Kazuhiro, Nakatado-gun, Kagawa 764-0026 (JP); MATSUOKA, Shoji, Nakatado-gun, Kagawa 764-0026 (JP)
(74) Representative: Gulde & Partner
(86) International application number: PCT/JP2019/000621
(87) International publication number: WO 2019/230035

(57) **Abstract**

A multi-layer culture vessel observation system, a cart device, and a multi-layer culture vessel observation device that enable an operator to easily observe objects to be observed in a multi-layer culture vessel are provided. A multi-layer culture vessel observation system includes: a cart device 20 capable of carrying a multi-layer culture vessel 30 that includes a plurality of trays and moving; and an observation device 10 capable of observing objects to be observed in each tray in the multi-layer culture vessel 30, wherein the cart device 20 includes a frame body including a sideways exposure part that exposes two side faces of the multi-layer culture vessel 30 from an upper end to a lower end, wherein the observation device 10 includes: a storage 14 that stores the cart device 20 in a state of carrying the multi-layer culture vessel 30; and an imaging device 11 that includes an optical system and outputs an image formed by the optical system, and wherein, when the cart device 20 carrying the multi-layer culture vessel 30 is stored in the storage 14, the sideways exposure part is located on an optical axis of the imaging device 11.

## Description

### TECHNICAL FIELD

The present invention relates to a multi-layer culture vessel observation system, a cart device, and a multi-layer culture vessel observation device for observing objects to be observed in a multi-layer culture vessel including a plurality of trays.

### BACKGROUND ART

Multi-layer culture vessel observation devices for observing objects to be observed (culture) in a multi-layer culture vessel including a plurality of trays are known. As such a multi-layer culture vessel observation device, a culture vessel observation device in which an optical observation means facing obliquely upward and a lighting means facing obliquely downward are located facing each other with a multi-layer culture vessel therebetween to arrange the optical observation means, the multi-layer culture vessel, and the lighting means on the same optical axis in order to observe the state of cultured cells in each tray is known (for example, see Patent Literature (PTL) 1).

### Citation List

### Patent Literature

PTL 1: Japanese Patent No. 4049263

### SUMMARY

### Technical Problem

Conventionally, in the case of observing objects to be observed in a multi-layer culture vessel by a culture vessel observation device, an operator needs to carry a plurality of multi-layer culture vessels to the vicinity of the culture vessel observation device using a cart, unload a multi-layer culture vessel containing a culture solution from the cart, and then place the multi-layer culture vessel on a pedestal which is an observation position of the culture vessel observation device, to observe objects to be observed in the multi-layer culture vessel. Particularly in the case of observing objects to be observed in each of a plurality of multi-layer culture vessels, the operator needs to load and unload the multi-layer culture vessels containing culture solutions one by one. This increases the labor of the operator. In addition, since the operator manually handles the multi-layer culture vessel, the operator may give an unnecessary impact to the culture medium in the multi-layer culture vessel, or unnecessarily touch the multi-layer culture vessel and consequently damage the multi-layer culture vessel and cause contamination.

The present invention provides a multi-layer culture vessel observation system, a cart device, and a multi-layer culture vessel observation device that enable an operator to easily observe objects to be observed in a multi-layer culture vessel.

### Solution to Problem

A culture vessel observation system according to the present invention is a multi-layer culture vessel observation system including: a cart device capable of carrying a multi-layer culture vessel that includes a plurality of trays and moving; and an observation device capable of observing objects to be observed in each tray in the multi-layer culture vessel, wherein the cart device includes a frame body including a sideways exposure part that exposes two side faces of the multi-layer culture vessel from an upper end to a lower end, wherein the observation device includes: a storage that stores the cart device in a state of carrying the multi-layer culture vessel; and an imaging device that includes an optical system and outputs an image formed by the optical system, and wherein, when the cart device carrying the multi-layer culture vessel is stored in the storage, the sideways exposure part is located on an optical axis of the imaging device.

The culture vessel observation system may further include an operation device that holds and handles the multi-layer culture vessel, wherein the cart device includes: a cart having wheels; and a fixing member that is removably mounted on the cart, and fixes the multi-layer culture vessel to the cart, and wherein the operation device holds and handles the multi-layer culture vessel together with the fixing member.

The culture vessel observation system may further include a lighting device that faces the imaging device, and irradiates the imaging device with light, wherein, when the cart device carrying the multi-layer culture vessel is stored in the storage, the imaging device, the multi-layer culture vessel, and the lighting device are located on the same optical axis, the multi-layer culture vessel being interposed between the imaging device and the lighting device.

The culture vessel observation system may further include a drive portion that drives the imaging device and the lighting device in a first direction and a second direction, wherein the cart has a mounting space in which a plurality of multi-layer culture vessels is mountable side by side in the first direction.

In the multi-layer culture vessel observation system, the imaging device and the lighting device may be arranged so that the optical axis of the imaging device and the lighting device intersects a bottom of the tray in the multi-layer culture vessel at an angle in a range of 40° to 50°.

A cart device according to the present invention is a cart device storable in an observation device capable of observing objects to be observed in each of trays included in a multi-layer culture vessel, including a frame body that fixes the multi-layer culture vessel, wherein the frame body includes a sideways exposure part that exposes two side faces of the multi-layer culture vessel from an upper end to a lower end.

In the cart device, the frame body may comprise a plurality of frame bodies.

A multi-layer culture vessel observation device according to the present invention is a multi-layer culture vessel observation device including: a storage that stores the cart device described above in a state of carrying the multi-layer culture vessel; and an imaging device that includes an optical system and outputs an image formed by the optical system, wherein, when the cart device carrying the multi-layer culture vessel is stored in the storage, the sideways exposure part is located on an optical axis of the imaging device.

### Advantageous Effects of Invention

According to the present invention, it is possible to provide a multi-layer culture vessel observation system, a cart device, and a multi-layer culture vessel observation device that enable an operator to easily observe objects to be observed in a multi-layer culture vessel.

### BRIEF DESCRIPTION OF DRAWINGS

Fig. 1 is a diagram for explaining a multi-layer culture vessel according to an embodiment.
Fig. 2 is a diagram for explaining a method of distributing a liquid to each tray in the multi-layer culture vessel.
Fig. 3 is a perspective diagram illustrating a multi-layer culture vessel observation system according to Embodiment 1.
Fig. 4 is a perspective diagram illustrating a cart device according to the embodiment.
Fig. 5 is a perspective diagram illustrating an observation state of the cart device according to the embodiment.
Fig. 6 is a diagram in which (A) illustrates a lateral frame member according to the embodiment, (B) illustrates the cart device before the lateral frame member is attached and (C) illustrates the cart device to which the lateral frame member is attached.
Fig. 7 is a block diagram illustrating a multi-layer culture observation device according to the embodiment.
Fig. 8 is a diagram for explaining the positional relationship among an imaging device, the multi-layer culture vessel, and a lighting device.
Fig. 9 is a perspective diagram for explaining a multi-layer culture vessel operation device.
Fig. 10 is a block diagram illustrating the multi-layer culture vessel operation device.
Fig. 11 is a diagram for explaining the relationship between a latched portion of a fixing member and a latching member of the multi-layer culture vessel operation device.
Fig. 12 is a perspective diagram illustrating a state in which the multi-layer culture vessel is provisionally fixed to arms of the multi-layer culture vessel operation device.
Fig. 13 is a diagram illustrating an example of a rotation operation of a rotation portion around a rotation axis X1.
Fig. 14 is a diagram illustrating an example of a rotation operation of the rotation portion around a rotation axis X2 and a rotation operation of the rotation portion around the rotation axes X1 and X2.
Fig. 15 is a perspective diagram illustrating a state in which a shaking portion of the multi-layer culture vessel operation device is protruded.
Fig. 16 is a diagram for explaining the relationship between the arms and insertion holes in a shaking operation.
Fig. 17 is a block diagram for explaining a multi-layer culture vessel operation device according to Embodiment 3.

### DESCRIPTION OF EMBODIMENTS

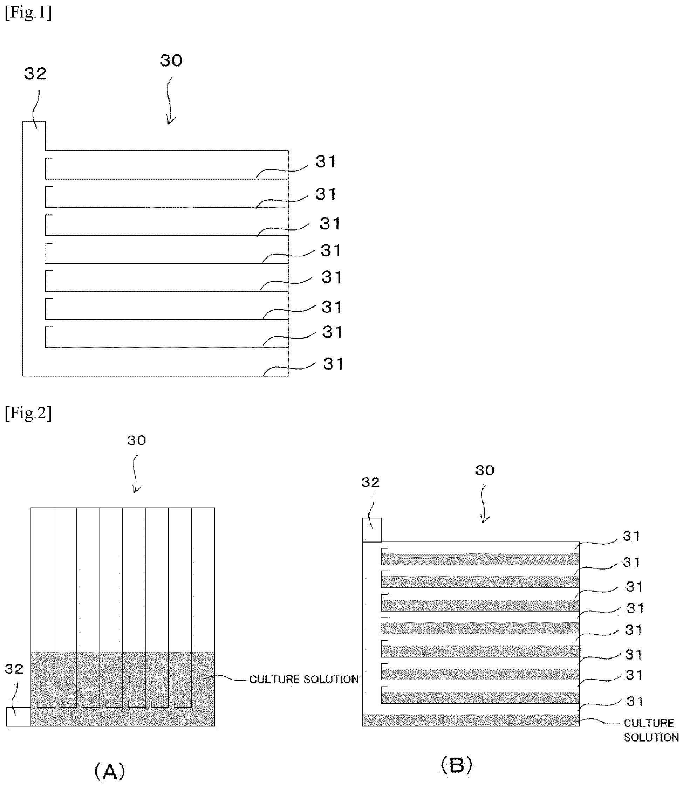

A multi-layer culture vessel observation system 1 according to an embodiment will be described below. The multi-layer culture vessel observation system 1 according to this embodiment is a system for observing cultured cells in a multi-layer culture vessel 30 used in cell culture and the like. The multi-layer culture vessel 30 according to this embodiment will be described first. Fig. 1 is a diagram for explaining the multi-layer culture vessel 30 according to this embodiment, and is a sectional diagram illustrating the multi-layer culture vessel 30. The multi-layer culture vessel 30 has a structure in which a plurality of trays 31 are stacked to culture cells in large quantity, as illustrated in Fig. 1. In the case of culturing cells in the multi-layer culture vessel 30, for example as illustrated in (A) in Fig. 2, the multi-layer culture vessel 30 is tilted about 90° so that a vent cap 32 will be on the lower side. The vent cap 32 and a pump are then connected, and a culture solution seeded with the cells is introduced into the multi-layer culture vessel 30 by the pump. After this, as illustrated in (B) in Fig. 2, the multi-layer culture vessel 30 is returned to the upright position, as a result of which the culture solution is distributed to each tray 31 in the multi-layer culture vessel 30. Thus, cell culture is performed in each tray 31.

### «Embodiment 1»

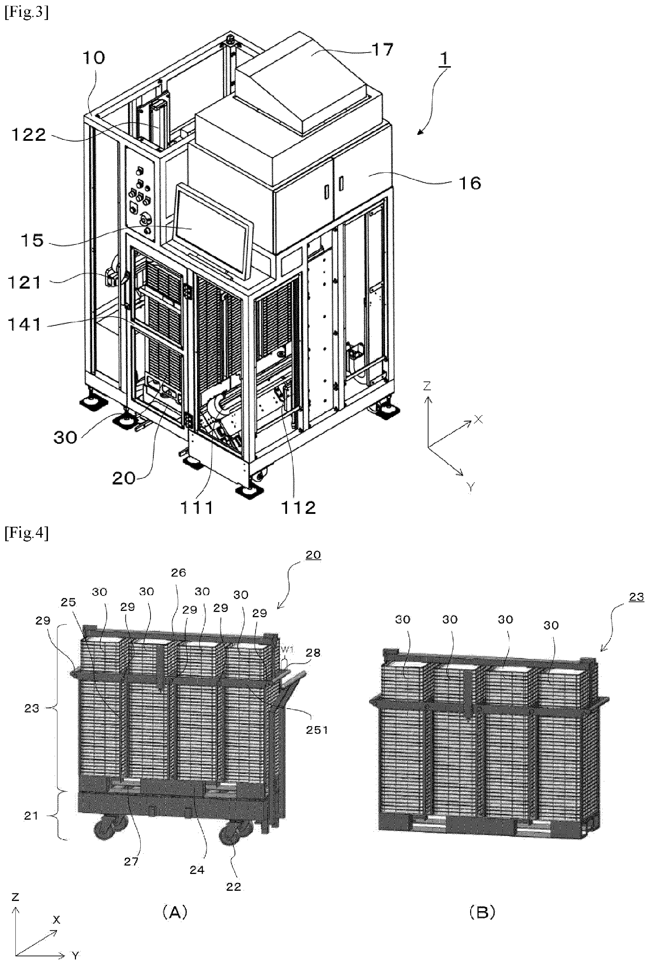

To observe cultured cells in such a multi-layer culture vessel 30, the multi-layer culture vessel observation system 1 according to this embodiment includes a multi-layer culture vessel observation device 10 and a cart device 20, as illustrated in Fig. 3. Fig. 3 is a perspective diagram illustrating the multi-layer culture vessel observation system 1. Fig. 3 illustrates a state in which the cart device 20 carrying the multi-layer culture vessel 30 has been moved to the inside of the multi-layer culture vessel observation device 10 which is an observation position. Each of the components will be described below.

Fig. 4 is a perspective diagram for explaining the cart device 20 according to this embodiment. As illustrated in (A) in Fig. 4, the cart device 20 includes a cart 21 having wheels 22, and a fixing member 23 that fixes the multi-layer culture vessel 30 to the cart 21. In the cart device 20, the cart 21 and the fixing member 23 are removably attachable to each other. Specifically, by lifting the fixing member 23 upward (Z-axis positive direction) with respect to the cart 21, the fixing member 23 can be removed from the cart 21, as illustrated in (B) in Fig. 4. By placing the fixing member 23 on the cart 21, the fixing member 23 can be attached to the cart 21. The cart 21 and the fixing member 23 have respective fitting portions (not illustrated) that fit together. Thus, the cart 21 and the fixing member 23 are removably attachable to each other only in the vertical direction (Z-axis direction), and are fixed in the horizontal direction (XY-axis direction). This can prevent the fixing member 23 from falling from the cart 21 during movement of the cart device 20.

The fixing member 23 can simultaneously fix a plurality of multi-layer culture vessels 30 in a state in which the plurality of multi-layer culture vessels 30 are arranged side by side, as illustrated in Fig. 4. In this embodiment, the cart device 20 is configured to carry four multi-layer culture vessels 30 at the maximum. However, the present invention is not limited to this. The cart device 20 may be configured to carry one to three multi-layer culture vessels 30 at the maximum, or configured to carry five or more multi-layer culture vessels 30.

As illustrated in (A) in Fig. 4, the fixing member 23 includes a pedestal 24 on which the multi-layer culture vessel 30 is placed, a frame member 25 that guards the four long sides of the multi-layer culture vessel 30 to keep the multi-layer culture vessel 30 from being misaligned in the horizontal direction (XY-axis direction), and a fastening member 26 that is latched to the frame member 25 and, when the multi-layer culture vessel 30 is rotated, guards the multi-layer culture vessel 30 so as not to project upward (Z-axis direction). For example, the operator can fix the multi-layer culture vessel 30 to the fixing member 23, by placing the multi-layer culture vessel 30 on the pedestal 24 within the frame member 25 and then latching the fastening member 26 and the frame member 25 together by pressing the upper side of the multi-layer culture vessel 30 with the fastening member 26.

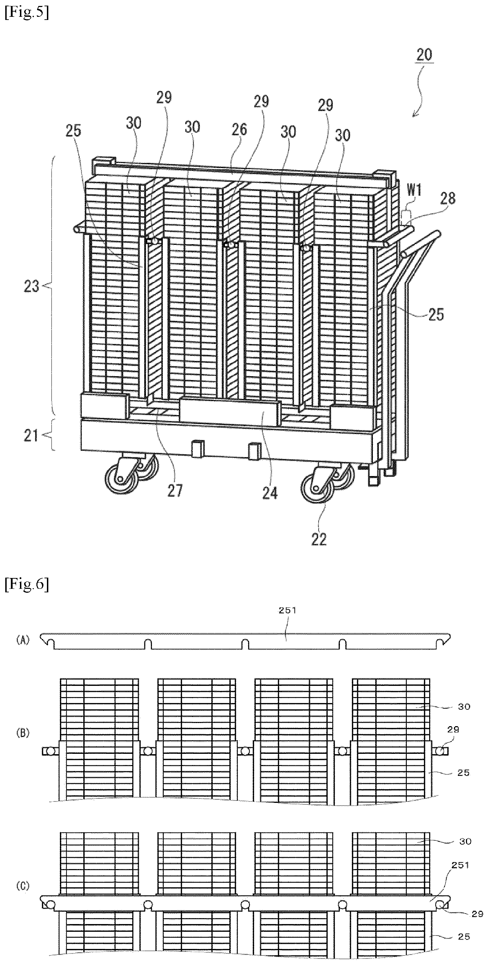

Fig. 5 is a perspective diagram illustrating an observation state of the cart device 20 according to this embodiment. Fig. 6 is a diagram for explaining a method of attaching and removing lateral frame members 251. By loosening fasteners 29 such as screws (or by removing the fasteners 29) in the cart device 20 illustrated in Fig. 4, a pair of lateral frame members 251 forming part of the frame member 25 can be removed from the fixing member 23 as illustrated in Fig. 5. Thus, the frame member 25 includes a sideways exposure part that exposes part of the side faces of the cart device 20 from the upper end to the lower end. Hence, in the case of observing the culture state in the multi-layer culture vessel 30 by the multi-layer culture vessel observation device 10, it is possible to effectively prevent a situation in which the observation of the multi-layer culture vessel 30 is obstructed by the lateral frame members 251. In the case of operating the multi-layer culture vessel 30 using the below-described multi-layer culture vessel operation device 40, for safety it is preferable to latch the grooves of the lateral frame members 251 to the fasteners 29 and tighten the fasteners 29 to thereby attach the pair of lateral frame members 251 to the fixing member 23, as illustrated in (A) to (C) in Fig. 6. Thus, the pair of lateral frame members 251 are attachable to and removable from the frame member 25.

The lateral frame members 251 can fix the multi-layer culture vessel 30 by being attached to the frame member 25, as illustrated in Fig. 4. However, even when the lateral frame members 251 are not attached to the frame member 25, the remaining parts of the frame member 25 (the frame member 25 extending in the direction in which the multi-layer culture vessels 30 are stacked) fix the four corners of the multi-layer culture vessel 30, so that it is possible to effectively prevent the multi-layer culture vessel 30 from falling from the cart device 20 when observing the cultured tissues in the multi-layer culture vessel 30 by the multi-layer culture vessel observation device 10.

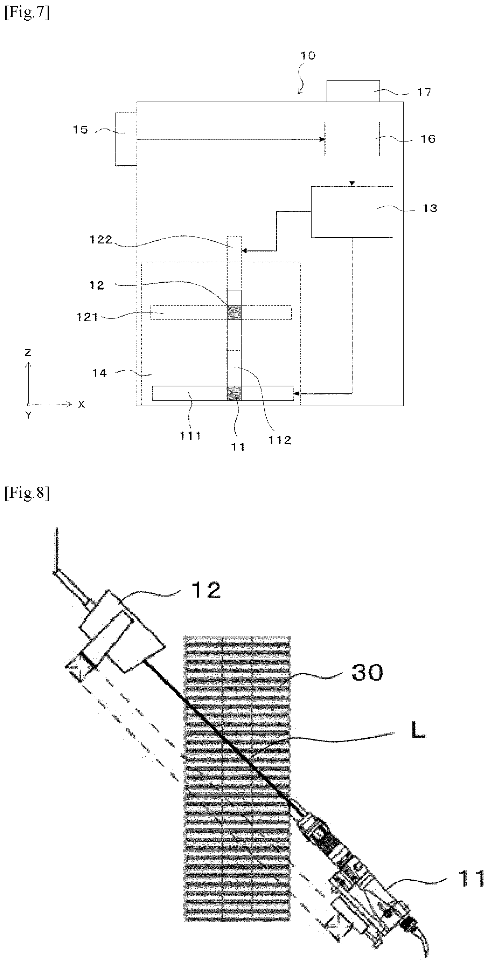

The multi-layer culture vessel observation device 10 according to this embodiment will be described below. Fig. 7 is a block diagram illustrating the multi-layer culture vessel observation device 10 according to this embodiment. As illustrated in Figs. 3 and 7, the multi-layer culture vessel observation device 10 includes an imaging device 11, a lighting device 12, first to fourth frames 111,112,121, and 122, a drive portion 13, a storage 14, a touch panel portion 15, a control portion 16, and a fan 17.

The imaging device 11 is a camera that includes a lens and focuses on the bottom of each tray 31 in the multi-layer culture vessel 30 and images cultured cells adhering to the bottom of the tray 31. The imaging device 11 is not limited as long as it is a camera, but preferably includes a zoom lens and is capable of capturing an image of each tray 31 with different magnification. In this embodiment, the imaging device 11 engages with the first frame 111 extending in the X-axis direction, and can be moved in the X-axis direction along the first frame 111 by the drive portion 13. The first frame 111 engages with the second frame 112 extending in the Z-axis direction, and can be moved in the Z-axis direction along the second frame 112 by the drive portion 13, as a result of which the imaging device 11 can be moved in the Z-axis direction, too. Thus, the imaging device 11 can be moved in a two-dimensional direction (XZ-axis direction) along the first frame 111 and the second frame 112 by the drive portion 13. The drive portion 13 includes an electric motor and/or an air cylinder, and can drive the frames 111 to 114 by the electric motor and/or air cylinder.

The lighting device 12 is a lighting device that irradiates the imaging device 11 with light. The lighting device 12 is not limited, and may be, for example, a lighting device having LEDs as a light source. In this embodiment, the lighting device 12 engages with the third frame 121, and can be moved in the X-axis direction along the third frame 121 by the drive portion 13. The third frame 121 engages with the fourth frame 122, and can be moved in the Z-axis direction along the fourth frame 122 by the drive portion 13, as a result of which the lighting device 12 can be moved in the Z-axis direction, too. Thus, the lighting device 12 can be moved in the two-dimensional direction (XZ-axis direction) along the third frame 121 and the fourth frame 122 by the drive portion 13, as with the imaging device 11.

In this embodiment, when the cart device 20 carrying the multi-layer culture vessel 30 is stored in the storage 14, the imaging device 11 and the lighting device 12 are arranged facing each other with the multi-layer culture vessel 30 therebetween, as illustrated in Fig. 8. Hence, in the example illustrated in Fig. 3, when the imaging device 11 is located on the right side (Y-axis positive side) of the multi-layer culture vessel 30, the lighting device 12 is located on the left side (Y-axis negative side) of the multi-layer culture vessel 30. Fig. 8 is a diagram for explaining the positional relationship among the imaging device, the multi-layer culture vessel, and the lighting device.

The imaging device 11 and the lighting device 12 are preferably arranged on the same optical axis L that passes the multi-layer culture vessel 30 obliquely, as illustrated in Fig. 8. It is preferable that the imaging device 11 faces obliquely upward and the lighting device 12 faces obliquely downward. The orientation of each of the imaging device 11 and the lighting device is adjustable. It is preferable to adjust the orientations of the imaging device 11 and the lighting device 12 so that the optical axis L intersects the bottom of the tray 31 interposed therebetween at an angle of approximately 45° (e.g. an angle of 40° to 50°).

In this embodiment, in the case where the cart device 20 carrying the multi-layer culture vessel 30 is stored in the storage 14, the cart device 20 is stored in the storage 14 with the lateral frame members 251 being removed from the frame member 25 as illustrated in Fig. 5. As a result of removing the lateral frame members 251 from the frame member 25, the lateral frame members do not exist on the optical axis of the imaging device 11 and the lighting device 12, with it being possible to appropriately observe the culture state of each layer in the multi-layer culture vessel 30.

The drive portion 13 moves the imaging device 11 and the lighting device 12 in the two-dimensional direction (XZ-axis direction). In particular, in this embodiment, the drive portion 13 moves the imaging device 11 and the lighting device 12 in the same direction by the same distance so that the imaging device 11 and the lighting device 12 will be located on the same optical axis L and the relative positions of the imaging device 11 and the lighting device 12 will be unchanged. For example, in this embodiment, the operator can operate the touch panel portion 15 to designate the position of the tray 31 to be observed, as described later. In the case where the operator operates the touch panel portion 15 to issue an instruction for movement of 5 mm in the X-axis positive direction, for example, the drive portion 13 drives the imaging device 11 to move 5 mm in the X-axis positive direction along the first frame 111, and drives the lighting device 12 to move 5 mm in the X-axis positive direction along the third frame 121. Moreover, the drive portion 13 can move the imaging device 11 and the lighting device 12 in the vertical direction (Z direction) to observe a tray 31 in a different layer (stage), and move the imaging device 11 and the lighting device 12 in the right-left direction (X direction) to observe a different multi-layer culture vessel 30.

The storage 14 is a space for storing the cart device 20 carrying the multi-layer culture vessel 30. The storage 14 includes a fixture for fixing the cart device 20 in a predetermined observation position. Thus, the cart device 20 can be fixed in the observation position in the storage 14, and the imaging device 11 can image the cultured cells in each tray 31 in the multi-layer culture vessel 30. A door 141 is installed at the entrance of the storage 14.

The touch panel portion 15 functions as an input portion for the operator to input instructions, and also functions as a display portion for displaying images captured by the imaging device 11. In this embodiment, the display region of the display of the touch panel portion 15 is divided between a display region for input and a display region for captured image display, and a captured image is displayed in the display region for captured image display and buttons (icons) for the operator to input instructions are displayed in the display region for input. The operator can designate the position of imaging by the imaging device 11 or the like, by pressing a corresponding button (icon) displayed in the display region for input.

The control portion 16 includes read only memory (ROM) that stores a program, a central processing unit (CPU) that executes the program stored in the ROM, and random access memory (RAM) that functions as an accessible storage device. Based on the program stored beforehand, the control portion 16 displays an image captured by the imaging device 11 on the touch panel portion 15 and outputs a drive instruction to the drive portion 13 based on an instruction input to the touch panel portion 15 by the operator. The fan 17 is provided above the control portion 16. The fan 17 is installed to perform suction for the control portion 16, as a result of which the control portion 16 can be cooled. Since the multi-layer culture vessel observation device 10 according to this embodiment is often used at relatively high temperature (e.g. 37°C) suitable for culture, the provision of the fan 17 can reduce the possibility that the control portion 16 fails due to heat.

As described above, in the multi-layer culture vessel observation system according to Embodiment 1, the cultured cells in each tray 31 in each multi-layer culture vessel 30 can be observed in a state in which the cart device 20 carries the multi-layer culture vessel 30. Since there is no need to place the multi-layer culture vessels 30 in the dedicated observation position (pedestal) one by one as in the conventional techniques, the labor of the operator can be reduced. Moreover, the operator can be prevented from giving an unnecessary impact to the culture medium in the multi-layer culture vessel 30, and unnecessarily touching the multi-layer culture vessel 30. Furthermore, in the multi-layer culture vessel observation system 1 according to this embodiment, a plurality of multi-layer culture vessels 30 can be mounted on one cart device 20 simultaneously, so that the cultured cells in the trays 31 of the plurality of multi-layer culture vessels 30 can be observed at one time.

### «Embodiment 2»

A multi-layer culture vessel observation system 1a according to Embodiment 2 will be described below. The multi-layer culture vessel observation system 1a according to Embodiment 2 includes a multi-layer culture vessel operation device 40 for operating (handling) the multi-layer culture vessel 30, in addition to the structure of the multi-layer culture vessel observation system 1 according to Embodiment 1.

In detail, the system according to Embodiment 2 is configured to perform a series of operations of introducing a culture solution seeded with cells into the multi-layer culture vessel 30 using the multi-layer culture vessel operation device 40, culturing the cells in the multi-layer culture vessel 30 for a certain period of time, and then observing the cultured cells using the multi-layer culture vessel observation device 10, in a state in which the cart device 20 carries the multi-layer culture vessel 30. The multi-layer culture vessel operation device 40 will be described below.

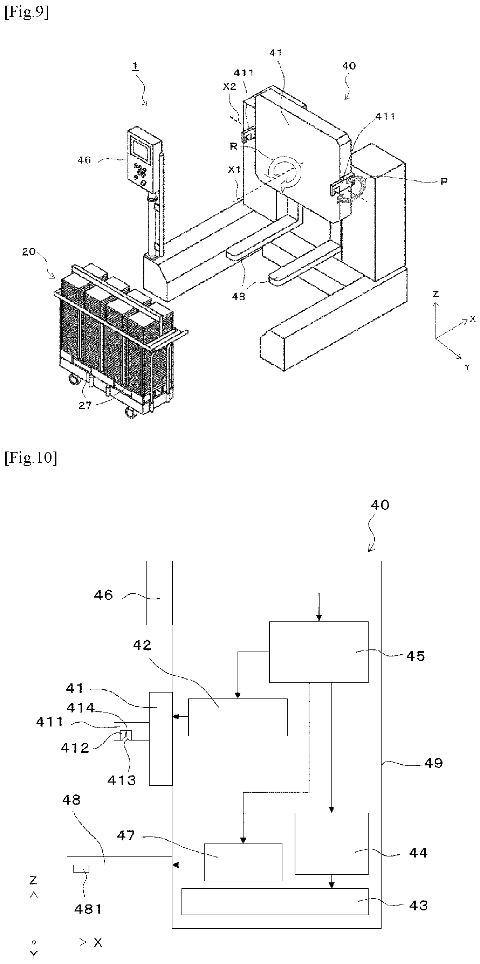

The multi-layer culture vessel operation device 40 is a device (manipulator) for operating the multi-layer culture vessel 30. Fig. 9 is a perspective diagram illustrating the multi-layer culture vessel operation device 40. Fig. 10 is a block diagram illustrating the multi-layer culture vessel operation device 40. As illustrated in Fig. 10, the multi-layer culture vessel operation device 40 includes a rotation portion 41, a rotation drive portion 42, a shaking portion 43, a shaking drive portion 44, a drive control portion 45, an operation portion 46, an arm drive portion 47, a pair of arms 48, and a body 49. The drive control portion 45 stores beforehand an operation program for operating the multi-layer culture vessel 30 in a culture solution introduction process of introducing a culture solution seeded with cells into the multi-layer culture vessel 30, a culture solution recovery process of recovering the culture solution from the multi-layer culture vessel 30, a trypsin introduction process of introducing a trypsin solution into the multi-layer culture vessel 30, a cell detachment process of shaking the multi-layer culture vessel 30, a trypsin solution recovery process of recovering the trypsin solution from the multi-layer culture vessel 30, and the like, and drives the rotation drive portion 42 and the shaking drive portion 44 based on the operation program. Specifically, based on the program, the drive control portion 45 controls the operation of the rotation drive portion 42 to cause the rotation drive portion 42 to rotate the rotation portion 41, and controls the operation of the shaking drive portion 44 to cause the shaking drive portion 44 to shake the shaking portion 43. In this embodiment, the rotation drive portion 42 and the shaking drive portion 44 each include an electric motor, and respectively drive the rotation portion 41 and the shaking portion 43 with supply of electric power.

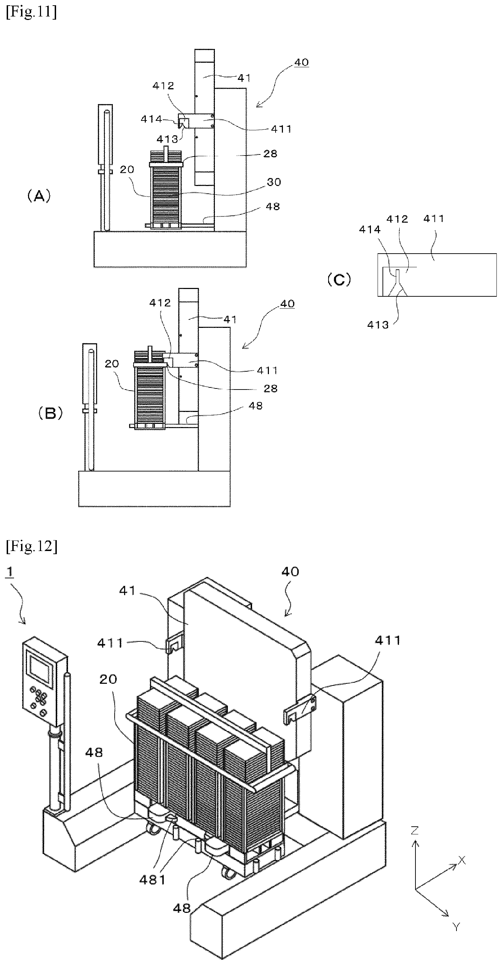

As illustrated in Fig. 9, the rotation portion 41 includes a pair of latching members 411 that function as a holding member for holding the multi-layer culture vessel 30. The latching members 411 are fixed on both sides of the rotation portion 41, and each have a recess 412 as illustrated in Fig. 10 and (A) to (C) in Fig. 11. The recess 412 has a tapered portion 413 and a groove portion 414. When the fixing member 23 is moved upward by the arm 48, the recess 412 can latch a latched portion 28 of the fixing member 23 and clamp the fixing member 23 with the arm 48 to fix the fixing member 23 to the rotation portion 41, as illustrated in (B) in Fig. 11. This will be described later. (A) and (B) in Fig. 11 are diagrams for explaining the relationship between the latched portion 28 and the latching member 411. (C) in Fig. 11 is an enlarged view of the latching member 411. In this embodiment, the latched portion 28 of the fixing member 23 is formed continuously in a part of the frame member 25 that extends along the side face of the multi-layer culture vessel 30 in the arrangement direction of the multi-layer culture vessels 30, and is a thin plate-like member having a length W1, as illustrated in (A) in Fig. 4.

The multi-layer culture vessel operation device 40 includes the pair of arms 48 that function as a support member for supporting the multi-layer culture vessel 30. The pair of arms 48 are insertable through two insertion holes 27 formed in the fixing member 23, as illustrated in Fig. 12. Fig. 12 is a perspective diagram illustrating a state in which the multi-layer culture vessel 30 is provisionally fixed to the arms 48. The pair of arms 48 are movable in the vertical direction (Z-axis direction) by the arm drive portion 47. The arm drive portion 47 drives the two arms 48 in the Z-axis direction to a height position at which the arms 48 can be inserted into the insertion holes 27 of the fixing member 23, based on an instruction by the drive control portion 45. Thus, the operator can move the cart device 20 toward the body 49 and insert the two arms 48 into the two insertion holes 27 of the fixing member 23. A clamp 481 is stored on the side of a tip part of each arm 48. When the arm 48 is inserted through the insertion hole 27, the clamp 481 protrudes from the side of the tip part of the arm 48 that has passed through the insertion hole 27. The drive control portion 45 then causes the arm drive portion 47 to drive the arms 48 upward (Z-axis positive direction) in a state in which the two arms 48 are inserted through the two insertion holes 27 of the fixing member 23, to lift the multi-layer culture vessel 30 to the position of the rotation portion 41. Consequently, the latching members 411 of the rotation portion 41 and the latched portion 28 of the fixing member 23 are latched together and the fixing member 23 is clamped by the rotation portion 41 with the pair of latching members 411 and the pair of arms 48, so that the multi-layer culture vessel 30 together with the fixing member 23 is fixed to the rotation portion 41, as illustrated in (B) in Fig. 11. In this embodiment, the arm drive portion 47 can drive the pair of arms 48 by an electric motor or an air cylinder.

The drive control portion 45 then causes the rotation drive portion 42 to perform a rotation operation of rotating the rotation portion 41 around two axes, i.e. rotation axes X1 and X2, as indicated by reference signs R and P in Fig. 9. As illustrated in Fig. 9, the rotation axis X1 is a rotation axis extending in the X-axis direction. Thus, the rotation portion 41 and the multi-layer culture vessel 30 held by the rotation portion 41 can be rotated in a roll direction R. The rotation axis X2 is a rotation axis extending in the Y-axis direction. Thus, the rotation portion 41 and the multi-layer culture vessel 30 held by the rotation portion 41 can be rotated in a pitch direction P. In the rotation operation, the rotation in the roll direction R is possible within a range of less than ±180°. In this embodiment, the rotation portion 41 can be rotated in the roll direction R within a range of ±120°. The rotation in the pitch direction P is possible within a range of less than ±180°, too. In this embodiment, the rotation portion 41 can be rotated in the pitch direction P within a range of ±30°. In this embodiment, the rotation drive portion 42 includes an electric motor and/or an air cylinder for rotating the rotation portion 41 around the rotation axis X1 and an electric motor and/or an air cylinder for rotating the rotation portion 41 around the rotation axis X2, and thus can rotate the rotation portion 41 around the two axes.

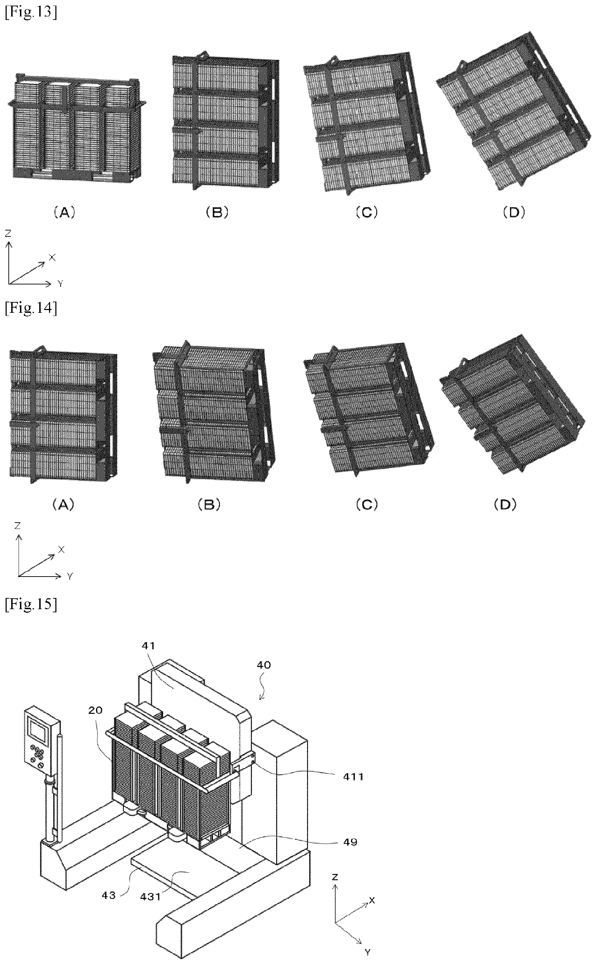

Fig. 13 is a diagram illustrating an example of a rotation operation of the rotation portion 41 (multi-layer culture vessel 30) around the rotation axis X1 by the rotation drive portion 42. (A) in Fig. 13 illustrates a state in which the rotation portion 41 has lifted the multi-layer culture vessel 30 (reference position). For example, the rotation drive portion 42 can rotate the rotation portion 41 (multi-layer culture vessel 30) 90° to the left around the rotation axis X1 from the reference position illustrated in (A) in Fig. 13, as illustrated in (B) in Fig. 13. Moreover, the rotation drive portion 42 can rotate the rotation portion 41 (multi-layer culture vessel 30) 100° to the left around the rotation axis X1 from the reference position as illustrated in (C) in Fig. 13, and can rotate the rotation portion 41 (multi-layer culture vessel 30) 120° to the left around the rotation axis X1 from the reference position as illustrated in (D) in Fig. 13. In this way, the rotation drive portion 42 can rotate the rotation portion 41 (multi-layer culture vessel 30) in the roll direction R within a range of ±0° to ±120° around the rotation axis X1 from the reference position.

Fig. 14 is a diagram illustrating an example of a rotation operation of the rotation portion 41 (multi-layer culture vessel 30) around the rotation axis X2 and a rotation operation of the rotation portion 41 (multi-layer culture vessel 30) around the two axes, i.e. the rotation axes X1 and X2. The rotation drive portion 42 can rotate the rotation portion 41 (multi-layer culture vessel 30) in the pitch direction P around the rotation axis X2 from the reference position illustrated in (A) in Fig. 14. For example, in the example illustrated in (B) in Fig. 14, the rotation portion 41 (multi-layer culture vessel 30) is rotated 20° around the rotation axis X2 so as to tilt an upper part of the rotation portion 41 (multi-layer culture vessel 30) forward (X-axis negative direction). The rotation drive portion 42 can also rotate the rotation portion 41 (multi-layer culture vessel 30) around the rotation axis X2 so as to tilt a lower part of the rotation portion 41 (multi-layer culture vessel 30) forward (X-axis negative direction). In this way, the rotation drive portion 42 can rotate the rotation portion 41 (multi-layer culture vessel 30) in the pitch direction P within a range of 0° to ±30° around the rotation axis X2 from the reference position.

The rotation drive portion 42 can rotate the rotation portion 41 (multi-layer culture vessel 30) in the roll direction R around the rotation axis X1 and in the pitch direction P around the rotation axis X2, as illustrated in (C) and (D) in Fig. 14. For example, in the example illustrated in (C) in Fig. 14, the rotation portion 41 (multi-layer culture vessel 30) is rotated 100° to the left around the rotation axis X1, and also rotated 20° around the rotation axis X2 so as to tilt the upper part of the rotation portion 41 (multi-layer culture vessel 30) forward (X-axis negative direction). In the example illustrated in (D) in Fig. 14, the rotation portion 41 (multi-layer culture vessel 30) is rotated 120° to the left around the rotation axis X1, and also rotated 20° around the rotation axis X2 so as to tilt the upper part of the rotation portion 41 (multi-layer culture vessel 30) forward (X-axis negative direction).

The rotation drive portion 42 can also perform a swinging operation of reciprocatingly rotating the rotation portion 41 (multi-layer culture vessel 30) around the rotation axis X1 or the rotation axis X2. For example, the rotation drive portion 42 can perform a swinging operation around the rotation axis X1, by reciprocatingly rotating the rotation portion 41 (multi-layer culture vessel 30) in the roll direction R within a range of ±120° around the rotation axis X1. The rotation drive portion 42 can also perform a swinging operation around the rotation axis X2, by reciprocatingly rotating the rotation portion 41 (multi-layer culture vessel 30) in the pitch direction P within a range of ±20° around the rotation axis X2 so as to tilt the upper part of the rotation portion 41 (multi-layer culture vessel 30) forward (X-axis negative direction) and then tilt the lower part of the rotation portion 41 (multi-layer culture vessel 30) forward (X-axis negative direction).

The shaking portion 43 is stored inside the body 49 of the multi-layer culture vessel operation device 40 when not performing the below-described shaking operation. When performing the shaking operation, the shaking portion 43 is protruded to the outside of the body 49 by the shaking drive portion 44 under control of the drive control portion 45, as illustrated in Fig. 15. Fig. 15 is a perspective diagram illustrating a state in which the shaking portion 43 is protruded from the multi-layer culture vessel operation device 40. The shaking portion 43 has an upper surface 431 wide enough to have the multi-layer culture vessel 30 together with the fixing member 23 placed thereon, and can perform a shaking operation in the horizontal direction with the multi-layer culture vessel 30 together with the fixing member 23 being placed on the upper surface 431. The shaking portion 43 also has a fitting portion (not illustrated) into which the fixing member 23 is to be fitted, same as the cart 21. Thus, the shaking portion 43 is removably attachable to the fixing member 23 in the vertical direction (Z-axis direction), while restricting the movement of the fixing member 23 in the horizontal direction (XY-axis direction) to prevent the fixing member 23 from falling from the shaking portion 43. The shaking drive portion 44 can cause the shaking portion 43 to perform any shaking operation as long as it is in the horizontal direction (XY-axis direction). For example, the shaking drive portion 44 may cause the shaking portion 43 to perform various shaking operations, such as a shaking operation from side to side (reciprocation operation in the Y-axis direction), a shaking operation back and forth (reciprocation operation in the X-axis direction), and a shaking operation in a figure-of-eight direction (shaking operation in a combination of the X-axis direction and the Y-axis direction).

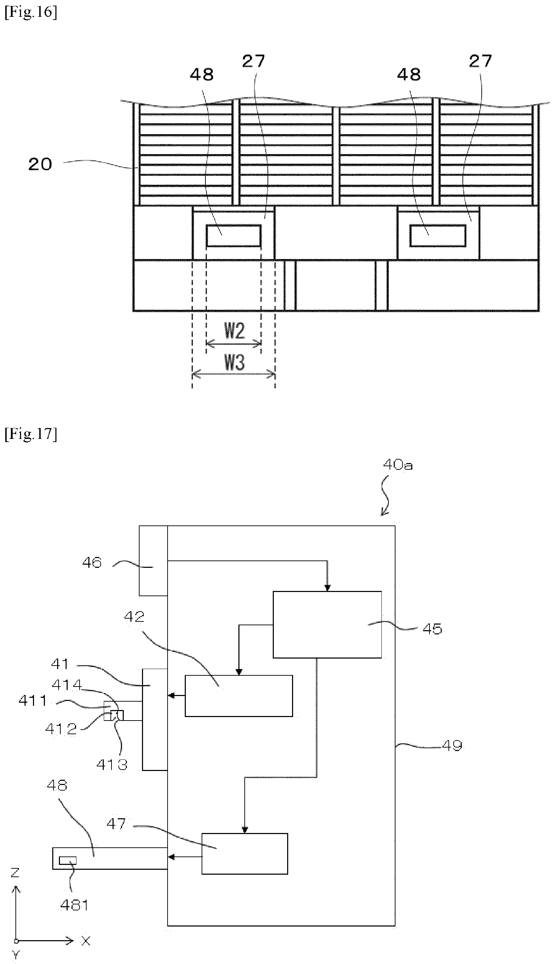

In this embodiment, the drive control portion 45 can cause the shaking portion 43 to perform the shaking operation following the rotation operation by the rotation portion 41. In this case, after the rotation operation by the rotation portion 41 ends, the drive control portion 45 controls the arm drive portion 47 to drive the pair of arms 48 downward (Z-axis negative direction) so that the multi-layer culture vessel 30 together with the fixing member 23 will be placed on the upper surface 431 of the shaking portion 43. The drive control portion 45 then controls the shaking drive portion 44 to cause the shaking portion 43 to perform the shaking operation of shaking the multi-layer culture vessel 30. In this embodiment, the shaking operation is performed in a state in which the arms 48 remain protruding without being stored in the body 49. In this embodiment, the inner width W3 of the insertion hole 27 is greater than the sum of the outer width W2 of the arm 48 and the shaking width of the shaking portion 43 as illustrated in Fig. 16, so that the shaking operation can be performed in a state in which the arm 48 is inserted through the insertion hole 27. In the shaking operation, the clamp 481 is stored inside the arm 48. Fig. 16 is a diagram for explaining the relationship between the arms 48 and the insertion holes 27 in the shaking operation.

The drive control portion 45 can cause the rotation portion 41 to perform the rotation operation following the shaking operation by the shaking portion 43. In this case, after the shaking operation by the shaking portion 43 ends, the drive control portion 45 controls the arm drive portion 47 to drive the pair of arms 48 upward (Z-axis positive direction) so that the multi-layer culture vessel 30 together with the fixing member 23 will be lifted upward. The drive control portion 45 then controls the shaking drive portion 44 to store the shaking portion 43 inside the body 49, and subsequently controls the rotation drive portion 42 to cause the rotation portion 41 to perform the rotation operation.

As mentioned above, in the case of operating the cart device 20 carrying the multi-layer culture vessel 30 by the multi-layer culture vessel operation device 40, it is preferable to attach the lateral frame members 251 to the frame member 25 as illustrated in Fig. 4. This makes it possible to operate the multi-layer culture vessel 30 by the multi-layer culture vessel operation device 40 more safely.

The operation portion 46 is a device for the operator to input instructions, and may include, for example, a touch panel. By operating the operation portion 46, the operator can transmit, to the drive control portion 45, an instruction such as starting, halting, or ending the program stored in the drive control portion 45 beforehand, to start, halt, or end the operation of the rotation portion 41 or the shaking portion 43, for example. Moreover, by operating the operation portion 46, the operator can cause the drive control portion 45 to store a new program or partially change the stored program. For example, by operating the operation portion 46, the operator can change the inclination angle of the rotation axis X1 of the rotation portion 41 from 100° to 120° in the culture solution recovery process of recovering the culture solution from the multi-layer culture vessel 30.

Thus, the multi-layer culture vessel operation device 40 according to this embodiment lifts the fixing member 23 fixing the multi-layer culture vessel 30 from the cart device 20 and holds the fixing member 23 fixing the multi-layer culture vessel 30 and performs the rotation operation and the shaking operation of the multi-layer culture vessel 30. In this way, the series of processes, i.e. the introduction of the culture solution seeded with cells into the multi-layer culture vessel 30, and, after the cell culture, the recovery of the culture solution, the introduction of the trypsin solution into the multi-layer culture vessel 30, the cell detachment, and the recovery of the trypsin solution after the cell detachment, can be performed without the operator touching the multi-layer culture vessel 30. With the conventional techniques, after introducing the trypsin solution into the multi-layer culture vessel 30, the operator manually removes the multi-layer culture vessel 30 from the multi-layer culture vessel operation device, places the multi-layer culture vessel 30 on a dedicated shaker, shakes the multi-layer culture vessel 30 by the dedicated shaker, and then manually places the multi-layer culture vessel 30 on the multi-layer culture vessel operation device again to perform the trypsin solution recovery process. This increases the labor of the operator in the cell culture process. In addition, due to the manual operation by the operator, the operation may vary, or the operator may unnecessarily touch the multi-layer culture vessel 30 and consequently damage the multi-layer culture vessel and cause contamination. With the multi-layer culture vessel operation device 40 according to this embodiment, such problems can be solved because the foregoing series of processes can be performed without the operator touching the multi-layer culture vessel 30.

The multi-layer culture vessel observation system 1a according to Embodiment 2 includes the multi-layer culture vessel operation device 40, so that the series of processes, i.e. the introduction of the culture solution into the multi-layer culture vessel 30, the cell culture, the observation of the cultured cells, and the recovery of the cultured cells, can be performed without the operator touching the multi-layer culture vessel 30. That is, the operator can introduce the culture solution seeded with cells into the multi-layer culture vessel 30 using the multi-layer culture vessel operation device 40, and subsequently return the multi-layer culture vessel 30 to the cart device 20 directly from the multi-layer culture vessel operation device 40 and move the multi-layer culture vessel 30 to a culture chamber or the like by the cart device 20. Moreover, in the case of observing the cultured cells, the operator can move the multi-layer culture vessel 30 mounted on the cart device 20 into the storage 14 of the multi-layer culture vessel observation device 10 and, in a state in which the cart device 20 carries the multi-layer culture vessel 30, observe the cultured cells using the multi-layer culture vessel observation device 10. Further, in the case where the operator determines that the cell culture is completed as a result of the observation, the operator can perform the culture solution recovery process, the trypsin solution introduction process, the cell detachment process, and the trypsin solution recovery process by the multi-layer culture vessel operation device 40, while the multi-layer culture vessel 30 is held by the multi-layer culture vessel operation device 40.

As described above, in the multi-layer culture vessel observation system 1a according to Embodiment 2, the multi-layer culture vessel 30 can be operated and observed without the operator touching the multi-layer culture vessel 30. Hence, the series of processes, i.e. the introduction of the culture solution seeded with cells into the multi-layer culture vessel 30, the cell culture, the observation of the cultured cells, and the recovery of the cultured cells, can be performed without the operator touching the multi-layer culture vessel 30. This can reduce the labor of the operator in mass cell culture, and effectively prevent the cultured cells from being adversely affected as a result of the operator giving an unnecessary impact to the culture medium in the multi-layer culture vessel 30 or unnecessarily touching the multi-layer culture vessel 30.

### «Embodiment 3»

A multi-layer culture vessel observation system 1b according to Embodiment 3 mainly differs from the multi-layer culture vessel observation system 1a according to Embodiment 2 in that a multi-layer culture vessel operation device 40a does not include the shaking portion 43 and the shaking drive portion 44. Hereafter, the same components as in Embodiment 2 are given the same reference signs, and their description is omitted.

Fig. 17 is a block diagram illustrating the multi-layer culture vessel operation device 40a according to this embodiment. As illustrated in Fig. 17, the multi-layer culture vessel operation device 40a includes the rotation portion 41, the rotation drive portion 42, the drive control portion 45, the operation portion 46, the arm drive portion 47, the pair of arms 48, and the body 49. The drive control portion 45 stores beforehand an operation program for operating the multi-layer culture vessel 30 in the culture solution introduction process of introducing the culture solution seeded with the cells into the multi-layer culture vessel 30, the culture solution recovery process of recovering the culture solution from the multi-layer culture vessel 30, the trypsin introduction process of introducing the trypsin solution into the multi-layer culture vessel 30, the cell detachment process of shaking the multi-layer culture vessel 30, the trypsin solution recovery process of recovering the trypsin solution from the multi-layer culture vessel 30, and the like.

The rotation drive portion 42 includes a first electric motor that rotates the rotation portion 41 around the rotation axis X1 and a second electric motor that rotates the rotation portion 41 around the rotation axis X2.

The operation program causes the cell detachment process to be performed by the rotation portion 41 reciprocatingly swinging the multi-layer culture vessel 30 in a first direction (e.g. to the right) and in a second direction (e.g. to the left) around a first rotation axis or a second rotation axis. The operation program defines a stop mode in which the movement of the multi-layer culture vessel 30 is stopped for a designated time when switching from the rotation operation in the first direction to the rotation operation in the second direction and when switching from the rotation operation in the second direction to the rotation operation in the first direction.

With the stop mode, even in the case where the speed of the swinging operation is higher than the speed of the movement of the liquid in the vessel, by stopping the swinging operation for the designated time when switching the direction of the rotation operation, the liquid in the vessel can reliably collide with the side surface (side wall) of the vessel. While it is important to swing the vessel at high speed for an effective cell detachment process, the delay (time lag) of the movement of the liquid which occurs when swinging the vessel at high speed can be resolved in this way.

The operation program causes an operation of recovering cells adhering to the side surface of each tray 31 to be performed before the trypsin solution recovery process. In the case where each tray 31 is a rectangular tray having first to fourth side surfaces, the rotation portion 41 is operated so that the trypsin solution will collide with the first side surface, the second side surface, the third side surface, and the fourth side surface in this order. With such operation, the trypsin solution recovery process can be performed after recovering, in the trypsin solution, the cells adhering to the side surface of each tray 31.

The multi-layer culture vessel operation device 40a according to Embodiment 3 described above has the same advantageous effects as in Embodiment 2.

Moreover, according to Embodiment 3, the cell detachment process is possible without the shaking portion 43. Since the device structure is simple, the manufacturing costs can be reduced.

Furthermore, the stop mode can resolve the delay (time lag) of the movement of the liquid which occurs when swinging the vessel at high speed.

While some preferred embodiments of the present invention have been described above, the technical scope of the present invention is not limited to the foregoing embodiments. Various modifications and improvements can be made to the foregoing embodiments, and such modifications and improvements are also included in the technical scope of the present invention.

For example, although the foregoing embodiments describe an example in which the multi-layer culture vessel observation device 10 includes the touch panel portion 15, the present invention is not limited to such. For example, the multi-layer culture vessel observation device 10 may separately include an input portion for the operator to input instructions and a display portion for displaying images captured by the imaging device 11.

Although the foregoing embodiments describe an example in which the multi-layer culture vessel observation systems 1 to 1b and the multi-layer culture vessel observation device 10 are used in observation of cultured cells, the present invention is not limited to such. The multi-layer culture vessel observation systems 1 to 1b and the multi-layer culture vessel observation device 10 may be used in observation of cultured microorganisms.

The multi-layer culture vessel observation device 10 may have any of the following structures, in addition to the foregoing embodiments.

In the case where the drive portion 13 is formed by an air cylinder, the multi-layer culture vessel observation device 10 may include a pressure sensor for monitoring the pressure of the air cylinder. By monitoring the air pressure of the air cylinder by the pressure sensor, a failure of the air cylinder can be detected.

The control portion 16 may be configured to count the number of times the drive portion 13 is operated. The control portion 16 may be configured to predict when to replace an electric motor or an air cylinder included in the drive portion 13, based on the number of times the drive portion 13 is operated.

The control portion 16 may be configured to measure the total operation time of the drive portion 13. The control portion 16 may be configured to predict when to replace an AC/DC power source, a battery, the fan 17, or the like, based on the total operation time of the drive portion 13.

The multi-layer culture vessel operation devices 40 and 40a may each have any of the following structures, in addition to Embodiments 2 and 3.

In the case where the rotation drive portion 42 and the arm drive portion 47 are formed by an air cylinder, the multi-layer culture vessel operation devices 40 and 40a may each include a pressure sensor for monitoring the pressure of the air cylinder. By monitoring the air pressure of the air cylinder by the pressure sensor, a failure of the air cylinder can be detected.

The multi-layer culture vessel operation devices 40 and 40a may each include an area sensor. By constantly monitoring, by the area sensor, whether anyone comes near the multi-layer culture vessel operation device and in particular the rotation portion 41, safety can be improved.

The drive control portion 45 may be configured to count the number of times the rotation portion 41, the shaking portion 43, or the arms 48 are operated. The drive control portion 45 may be configured to predict when to replace an electric motor or an air cylinder included in the rotation drive portion 42, the shaking drive portion 44, or the arm drive portion 47, based on the number of times the rotation portion 41, the shaking portion 43, or the arms 48 are operated.

The drive control portion 45 may be configured to measure the total operation time of the rotation portion 41, the shaking portion 43, or the arms 48. The drive control portion 45 may be configured to predict when to replace an AC/DC power source, a battery, a fan, or the like, based on the total operation time of the rotation portion 41, the shaking portion 43, or the arms 48.

Although the foregoing embodiments describe an example in which the cart device 20 includes a pair of lateral frame members 251, the present invention is not limited to such. For example, the cart device 20 may include a plurality of pairs of lateral frame members 251.

### Reference Signs List

- 1, 1a, 1b: multi-layer culture vessel observation system
- 10: multi-layer culture vessel observation device
- 11: imaging device
- 111: first frame
- 112: second frame
- 12: lighting device
- 121: third frame
- 122: fourth frame
- 13: drive portion
- 14: storage
- 141: door
- 15: touch panel portion
- 16: control portion
- 17: fan
- 20: cart device
- 21: cart
- 22: wheel
- 23: fixing member
- 24: pedestal
- 25: frame member
- 251: lateral frame member
- 26: fastening member
- 27: insertion hole
- 28: latched portion
- 29: fastener
- 30: multi-layer culture vessel
- 31: tray
- 32: vent cap
- 40, 40a: multi-layer culture vessel operation device
- 41: rotation portion
- 411: latch member
- 412: recess
- 413: tapered portion
- 414: groove portion
- 42: rotation drive portion
- 43: shaking portion
- 431: upper surface
- 44: shaking drive portion
- 45: drive control portion
- 46: operation portion
- 47: arm drive portion
- 48: arm
- 481: clamp
- 49: body

## Claims

1. A multi-layer culture vessel observation system comprising:
a cart device capable of carrying a multi-layer culture vessel that includes a plurality of trays and moving; and
an observation device capable of observing objects to be observed in each tray in the multi-layer culture vessel,
wherein the cart device includes a frame body including a sideways exposure part that exposes two side faces of the multi-layer culture vessel from an upper end to a lower end,
wherein the observation device includes:
a storage that stores the cart device in a state of carrying the multi-layer culture vessel; and
an imaging device that includes an optical system and outputs an image formed by the optical system, and
wherein, when the cart device carrying the multi-layer culture vessel is stored in the storage, the sideways exposure part is located on an optical axis of the imaging device.

2. The multi-layer culture vessel observation system according to claim 1, further comprising
an operation device that holds and handles the multi-layer culture vessel,
wherein the cart device includes:
a cart having wheels; and
a fixing member that is removably mounted on the cart, and fixes the multi-layer culture vessel to the cart, and
wherein the operation device holds and handles the multi-layer culture vessel together with the fixing member.

3. The multi-layer culture vessel observation system according to claim 1 or 2, further comprising
a lighting device that faces the imaging device, and irradiates the imaging device with light,
wherein, when the cart device carrying the multi-layer culture vessel is stored in the storage, the imaging device, the multi-layer culture vessel, and the lighting device are located on the same optical axis, the multi-layer culture vessel being interposed between the imaging device and the lighting device.

4. The multi-layer culture vessel observation system according to claim 3, further comprising
a drive portion that drives the imaging device and the lighting device in a first direction and a second direction,
wherein the cart has a mounting space in which a plurality of multi-layer culture vessels is mountable side by side in the first direction.

5. The multi-layer culture vessel observation system according to claim 3 or 4, wherein the imaging device and the lighting device are arranged so that the optical axis of the imaging device and the lighting device intersects a bottom of the tray in the multi-layer culture vessel at an angle in a range of 40° to 50°.

6. A cart device storable in an observation device capable of observing objects to be observed in each of trays included in a multi-layer culture vessel, the cart device comprising
a frame body that fixes the multi-layer culture vessel,
wherein the frame body includes a sideways exposure part that exposes two side faces of the multi-layer culture vessel from an upper end to a lower end.

7. The cart device according to claim 6, wherein the frame body comprises a plurality of frame bodies.

8. A multi-layer culture vessel observation device comprising:
a storage that stores the cart device according to claim 6 or 7 in a state of carrying the multi-layer culture vessel; and
an imaging device that includes an optical system and outputs an image formed by the optical system,
wherein, when the cart device carrying the multi-layer culture vessel is stored in the storage, the sideways exposure part is located on an optical axis of the imaging device.
